# EUROPEAN PATENT SPECIFICATION

(11) **EP 3 223 915 B1**
(45) Date of publication and mention of the grant of the patent: **24.02.2021**
(21) Application number: 14908726.4
(22) Date of filing: 24.12.2014
(51) Int. Cl.: A61Q 11/00, A61K 8/30, A61K 8/46, A61K 8/90, A61K 8/60, A61K 8/67

(54) **ORAL CARE COMPOSITION**
MUNDPFLEGEZUSAMMENSETZUNG
COMPOSITION DE SOINS BUCCO-DENTAIRES

(43) Date of publication of application: 04.10.2017
(73) Proprietor: Colgate-Palmolive Company, New York, NY 10022 (US)
(72) Inventor: XU, Yun, Guangzhou Guangdong 510730 (CN); LIN, Guangxin, Guangzhou Guangdong 510730 (CN); ZENG, Yuyan, Guangzhou Guangdong 510730 (CN); SONG, Wei, Guangzhou Guangdong 510730 (CN); LU, Hailiang, Guangzhou Guangdong 510730 (CN); CHEN, Dandan, Bridgewater, New Jersey 0880 (US); XU, Shao Peng, Guangzhou Guangdong (CN)
(74) Representative: Wibbelmann, Jobst
(86) International application number: PCT/CN2014/094755
(87) International publication number: WO 2016/101158

(56) References cited:
- WO-A1-95/19760
- WO-A1-2013/049507
- WO-A2-2008/042279
- WO-A2-2009/100283
- US-A- 5 275 805
- US-A1- 2004 067 204
- US-A1- 2006 141 072
- US-A1- 2007 292 461
- US-B1- 6 429 231
- US-B1- 6 692 726
- US-B1- 8 658 139

## Description

### BACKGROUND

The generation of free radicals in the oral cavity by oxidation reactions can irritate oral mucosal cells. Such oxidative irritation may in turn lead to the degradation of gum tissue and to periodontal disease. Antioxidants have been proposed as ingredients of oral care compositions such as dentifrices. The antioxidants inhibit irritating oxidation reactions that may occur in the oral cavity, thereby enhancing oral health.

It would be desirable to provide improved oral care formulations with enhanced antioxidative function.

WO 95/19760 (A1) discloses an oral composition having a reduced irritancy to oral tissue the composition containing a surfactant system comprised of an effective amount of an anionic surfactant and an alkyl glycoside having the formula RO (C₆H₁₀O₅)ₓ H, wherein R is an aliphatic residue of a C12 to C22 fatty alcohol and x is an integer from 1 to 20.

WO 2013/049507 (A1) discloses discloses an antioxidant composition which may include flavonoids such as rutin and may include surfactants.

US 5275805 (A) discloses an oral composition for inhibiting plaque on teeth comprising a vehicle in which is incorporated an antiplaque system of (a) an effective antibacterial amount of a salicylanilide compound and (b) a surfactant mixture of an anionic surfactant, a polyoxyethylene-polyoxypropylene block copolymer and a taurate salt, the pH of the composition being about 8.0 to 11.0.

### BRIEF SUMMARY

The present inventors have demonstrated that surfactants such as sodium lauryl sulfate surfactant, which are commonly used in oral care compositions (and in particular, dentifrice compositions) for foam production, cleaning and emulsification, may counteract the antioxidant benefit provided by the compositions. The present inventors have unexpectedly found that a surfactant system comprising the specific combination of sodium methyl cocoyl taurate and poloxamer may be used to replace conventional surfactants such as sodium lauryl sulfate surfactant, whilst maintaining foam production and cleansing activity without counteracting the antioxidant effect of the composition.

Accordingly, in a first aspect, there is provided an oral care composition comprising an antioxidant being rutin and a surfactant system, wherein the surfactant system consists of sodium methyl cocoyl taurate in an amount of from 0.8 weight % to 1 weight % by total weight of the composition and optionally, a poloxamer in an amount of from 0.4 to 1.2 weight % by total weight of the composition, and the composition does not comprise any surfactants other than those of the surfactant system. Preferably, the compositions comprises poloxamer.

Optionally, a further antioxidant can be selected from sodium ascorbyl phosphate, tocopheryl acetate, BeauPlex VH™ vitamin complex, quercetin catechin, trolox (6-hydroxy-2,5,7,8-tetramethylchroman-2-carboxylic acid), L-ascorbic acid, 2,4-dihydroxybenzoic acid, m-methoxybenzoic acid, m-hydroxybenzoic acid, p-hydroxybenzoic acid, 3,4-dihydroxybenzoic acid, stannous compounds, potassium or sodium meta-bisulfite, butylated hydroxytoluene (BHT), and ammonium sulfate

The antioxidant is rutin, which is present in the composition in an amount of from 0.2 weight % to 0.8 weight %, by total weight of the composition. More preferably, the rutin is present in the composition in an amount of 0.3 weight % to 0.5 weight %, by total weight of the composition.

The composition comprises sodium methyl cocoyl taurate in an amount of from 0.8 weight % to 1 weight % by total weight of the composition.

Optionally, the composition comprises poloxamer in an amount of from 0.4 weight % to 1.2 weight % by total weight of the composition. Further optionally, the composition comprises poloxamer in an amount of from 0.5 weight % to 0.8 weight % by total weight of the composition. Still further optionally, the composition comprises poloxamer in an amount of from 0.8 weight % to 1.2 weight % by total weight of the composition. Preferably, the poloxamer is poloxamer 407.

The surfactant system consists of sodium methyl cocoyl taurate and optionally, poloxamer, and the composition does not comprise any surfactants other than those of the surfactant system.

Optionally, the composition further comprises an agent selected from: desensitizing agents, abrasive agents, tartar control agents, binders, thickening agents, detergents, adhesion agents, foam modulators, pH modifying agents, mouth feel agents, sweeteners, flavorants, colorants, humectants, fluoride sources, whitening agents and combinations thereof.

Optionally, the composition is selected from mouthwashes, sprays, dentifrices, oral strips, chewing gums and lozenges. Preferably, the composition is a dentifrice. More preferably, the composition is a dentifrice composition comprising:
40 weight % to 60 weight % humectant, optionally glycerin;
0.1 weight % to 0.4 weight % cellulose binder, optionally sodium carboxymethyl cellulose;
0.05 weight % to 0.4 weight % sweetener, optionally sodium saccharin;
2 weight % to 4 weight % pH modifier, optionally citric acid monohydrate and trisodium citrate dehydrate;
15 weight % to 25 weight % abrasive silica;
1 weight % to 3 weight % thickener silica;
2 weight % to 4 weight % thickener, optionally Polyvinylpyrrolidone;
5 weight % to 10 weight % water;
0.8 weight % to 1.0 weight % sodium methyl cocoyl taurate;
and optionally, 0.5 weight % to 1 weight % poloxamer.

In a second aspect, there is provided an oral care composition comprising an antioxidant and a surfactant system, wherein the surfactant system comprises sodium methyl cocoyl taurate in an amount of from 0.8 weight % to 1 weight % by total weight of the composition and optionally, a poloxamer in an amount of from 0.4 to 1.2 weight % by total weight of the composition, and wherein the composition is for use in preventing irritation to oral mucosal cells.

Optionally, the composition is as defined herein. Further optionally, the use comprises preventing gingivitis and/or gum receding.

In a third aspect, there is provided an oral care composition, according to claim 7, comprising an antioxidant and a surfactant system, wherein the surfactant system comprises sodium methyl cocoyl taurate in an amount of from 0.8 weight % to 1 weight % by total weight of the composition and optionally, a poloxamer in an amount of from 0.4 to 1.2 weight % by total weight of the composition, and wherein the composition is for use in preventing periodontal disease.

Optionally, the composition is as defined herein. Further optionally, the use comprises preventing gingivitis and/or gum receding.

In a third aspect, there is provided a use of a surfactant system in an oral care composition comprising an antioxidant for preserving an antioxidant effect of the composition, wherein the surfactant system comprises sodium methyl cocoyl taurate and optionally, poloxamer, wherein the oral care composition comprises sodium methyl cocoyl taurate in an amount of from 0.8 weight % to 1 weight % by total weight of the composition, and optionally, poloxamer in an amount of from 0.4 to 1.2 weight % by total weight of the composition.

Optionally, the composition is as defined herein.

Optionally, the antioxidant effect of the composition comprises an oral mucosal cell protection effect.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

In one arrangement, there is provided an oral care composition comprising an antioxidant and a surfactant system, wherein the surfactant system comprises sodium methyl cocoyl taurate in an amount of from 0.7 weight % to 1.2 weight % by total weight of the composition and optionally, a poloxamer in an amount of from 0.4 to 1.2 weight % by total weight of the composition. Preferably, the compositions comprises poloxamer.

### Sodium methyl cocoyl taurate

Sodium methyl cocoyl taurate (sodium 2-(methylamino)ethanesulfonate) is preferably present in the composition in an amount of from 0.8 weight % to 1 weight % by total weight of the composition. In some embodiments, sodium methyl cocoyl taurate is present in the composition in an amount of from 0.9 weight % to 1 weight %, by total weight of the composition. In a preferred embodiment, sodium methyl cocoyl taurate is present in the composition in an amount of from 0.85 weight % to 1 weight %, by total weight of the composition. In a further embodiment, sodium methyl cocoyl taurate is present in the composition in an amount of from 0.87 weight % to 1 weight %, by total weight of the composition.

### Poloxamer

In one arrangement, the compositions provided herein comprise a poloxamer. Poloxamers are non-ionic triblock copolymers composed of a central hydrophobic chain of polyoxypropylene (poly(propylene oxide)) flanked by two hydrophilic chains of polyoxyethylene (poly(ethylene oxide)). Any poloxamer may be used in the compositions. Poloxamers that may be used in the compositions include without limitation, poloxamer 188, poloxamer 237, poloxamer 401 and poloxamer 407. In a preferred embodiment, the poloxamer is
poloxamer 407 (supplied by BASF as Pluronic™ F127 or by Croda as Synperonic™ PE/F 127). Poloxamer 407 has improved solubility in water and improved emulsification properties over other poloxamers.

The poloxamer may be present in the composition in an amount of from 0.4 weight % to 1.5 weight %, from 0.4 weight % to 1.4 weight %, or from 0.4 weight % to 1.3 weight %, or from 0.4 weight % to 1.2 weight %, by total weight of the composition. In some embodiments, the poloxamer is present in the composition in an amount of from 0.4 weight % to 1.1 weight %, from 0.4 weight % to 1 weight %, from 0.4 weight % to 0.9 weight %, from 0.4 weight % to 0.8 weight %, from 0.4 weight % to 0.7 weight %, or from 0.4 weight % to 0.6 weight %, by total weight of the composition. In other embodiments, the poloxamer is present in the composition in an amount of from 0.5 weight % to 1.5 weight %, from 0.5 weight % to 1.4 weight %, from 0.5 weight % to 1.3 weight %, from 0.5 weight % to 1.2 weight %, from 0.5 weight % to 1.1 weight %, from 0.5 weight % to 1 weight %, from 0.5 weight % to 0.9 weight %, from 0.5 weight % to 0.8 weight %, from 0.5 weight % to 0.7 weight %, or from 0.5 weight % to 0.6 weight %, by total weight of the composition. In further embodiments, the poloxamer is present in the composition in an amount of from 0.6 weight % to 1.5 weight %, from 0.6 weight % to 1.4 weight %, from 0.6 weight % to 1.3 weight %, from 0.6 weight % to 1.2 weight %, from 0.6 weight % to 1.1 weight %, from 0.6 weight % to 1 weight %, from 0.6 weight % to 0.9 weight %, from 0.6 weight % to 0.8 weight %, or from 0.6 weight % to 0.7 weight %, by total weight of the composition. In still further embodiments, the poloxamer is present in the composition in an amount of from 0.7 weight % to 1.5 weight %, from 0.7 weight % to 1,4 weight %, from 0.7 weight % to 1.3 weight %, from 0.7 weight % to 1.2 weight %, from 0.7 weight % to 1.1 weight %, from 0.7 weight % to 1 weight %, from 0.7 weight % to 0.9 weight %, from 0.7 weight % to 0.8 weight %, by total weight of the composition. In yet still further embodiments, the poloxamer is present in the composition in an amount of from 0.8 weight % to 1.5 weight %, from 0.8 weight % to 1.4 weight %, from 0.8 weight % to 1.3 weight %, from 0.8 weight % to 1.2 weight %, from 0.8 weight % to 1.1 weight %, or from 0.8 weight % to 1 weight %, by total weight of the composition. Optionally, the poloxamer is present in the composition in an amount of from 0.9 weight % to 1.5 weight %, from 0.9 weight % to 1.4 weight %, from 0.9 weight % to 1.3 weight %, from 0.9 weight % to 1.2 weight %, from 0.9 weight % to 1.1 weight %, or from 0.9 weight % to 1 weight %, by total weight of the composition. Preferably, the poloxamer is present in the composition in an amount of from 0.8 weight % to 1.4 weight %, or from 0.8 weight % to 1.2 weight %, or from 1 weight% to 1.2 weight % by total weight of the composition.

The surfactant system consists of the sodium methyl cocoyl taurate and optionally, poloxamer, and the composition does not comprise any surfactants other than those of the surfactant system. The present inventors have found that surfactants such as alkyl sulfates, alkyl ether sulfates, sarcosinates, carboxylated alkyl polyglycosides, fatty acyl glutamates, and alkyl glucosides have an irritating effect on the oral mucosa, thus counteracting the antioxidant benefit of the composition. Therefore, it is desirable to exclude such surfactants from the composition. Accordingly, in one arrangement, the composition provided herein is substantially free of one or more of: alkyl sulfates, alkyl ether sulfates, sarcosinates, alkyl glucose carboxylates, acyl glutamates, and alkyl glucosides. In other arrangements, the composition provided herein is substantially free of alkyl sulfates, alkyl ether sulfates, sarcosinates, alkyl glucose carboxylates, acyl glutamates, and alkyl glucosides. In a specific embodiment, the composition provided herein is substantially free of one or more surfactants selected from: sodium lauryl sulfate, sodium lauryl ether sulfate, sodium lauroyl sarcosinate, sodium lauryl glucose carboxylate, sodium cocoyl glutamate, and lauryl glucoside. In other embodiments, the composition provided herein is substantially free (of all of sodium lauryl sulfate, sodium lauryl ether sulfate, sodium lauroyl sarcosinate, sodium lauryl glucose carboxylate, sodium cocoyl glutamate, and lauryl glucoside

By "substantially free", it is meant that the relevant surfactant is present in an amount of 0.5 weight % or less, 0.4 weight % or less, 0.3 weight % or less, 0.2 weight % or less, or 0.1 weight % or less, by total weight of the composition. Accordingly, in some embodiments, the composition comprises one or more of: alkyl sulfates, alkyl ether sulfates, sarcosinates, alkyl glucose carboxylates, acyl glutamates, and alkyl glucosides, each in an amount of less than 0.5 weight %, by total weight of the composition. In other embodiments, the composition comprises one or more of: alkyl sulfates, alkyl ether sulfates, sarcosinates, alkyl glucose carboxylates, acyl glutamates, and alkyl glucosides, in a combined amount of less than 0.5 weight %, by total weight of the composition. In preferred embodiments, the composition does not comprise any alkyl sulfates, alkyl ether sulfates, sarcosinates, carboxylated alkyl polyglycosides, fatty acyl glutamates, and alkyl glucosides.

### Antioxidant

In one arrangement, the composition comprises an antioxidant. The term "antioxidant" as used herein means any agent having an antioxidant activity as determined by any generally accepted *in vitro* or *in vivo* antioxidant assay or test. Commonly used antioxidant test methods include free radical scavenging methods (for example, using 1,1-diphenyl-2-pierylhydrazyl (DPPH method), or 2,2'-azino-bis 3-ethylbenzthiazoline-6-sulfonic acid (ATBS methods), liquid peroxidation methods and antioxidant reduction methods.

Other optional antioxidants for use in the compositions provided herein include stannous compounds. Stannous compounds include organic and inorganic sources capable of releasing stannous ion. Non-limiting inorganic sources include stannous chloride, fluoride, sulfate, phosphate, metaphosphate, and pyrophosphate. Organic source include, without limitation, stannous acetate, tartrate, citrate, malate, malonate, gluconate, and oxalate. Further non-limiting examples of antioxidants include stannate materials such as potassium stannate; ammonium sulfate; phenolic antioxidants such as BHT; hydrioquinone antioxidants such as BHA; and sulfur containing antioxidants such as sodium or potassium metabisulfite.

The antioxidant is incorporated into the composition in a level effective to reduce or mitigate irritation to the oral cavity, and specifically to the oral mucosa. Without limitation, antioxidant levels in the composition may range from 0.01 weight % to 1 weight %m by total weight of the composition.

The present inventors have unexpectedly found that rutin has a stronger antioxidant activity than conventional antioxidants such as Vitamin E, in oral care formulations.Rutin is present in the composition in an amount of from 0.2 weight % to 0.8 weight %, or from 0.3 weight % to 0.6 weight %, by total weight of the composition. Preferably, rutin is present in the composition in an amount of from 0.2 weight % to 0.5 weight %, or from 0.3 weight % to 0.5 weight %, by total weight of the composition.

### Carrier and further optional ingredients

The compositions defined herein are suitable for application to the oral cavity and typically comprise an orally acceptable carrier. The expression "orally acceptable carrier" as used herein denotes any safe and acceptable materials for oral use. Such materials include water or other solvents that may contain a humectant such as glycerin, sorbitol, xylitol and the like. In some aspects, the term "orally acceptable carrier" encompasses all of the components of the oral care composition except for the antioxidant and surfactants of the surfactant system. In other aspects, the term refers to inert or inactive ingredients that serve to deliver the antioxidant and surfactants of the surfactant system, and/or any other functional ingredients, to the oral cavity.

Orally acceptable carriers for use in the invention include conventional and known carriers used in making mouthwashes or mouthrinses, dentifrices, tooth gels, tooth powder, lozenges, gums, beads, edible strips, tablets and the like. The compositions may be provided in such forms. Carriers should be selected for compatibility with each other and with other ingredients of the composition.

The oral care compositions of provided herein may further comprise additional ingredients. These additional ingredients may include, but are not limited to surfactants, diluents, pH modifying agents, other surfactants, desensitizing agents, tartar control agents, binders, thickening agents, detergents, adhesion agents, foam modulators, mouth feel agents, humectants, sweeteners, flavorants, colorants, antioxidants, sources of fluoride ions, whitening agents and mixtures thereof. Such ingredients and the amounts in which they could be incorporated would be known to those skilled in the art of oral care. However, non-limiting examples of these ingredients are provided below.

The compositions provided herein optionally incorporate one or more desensitizing agents. These include, without limitation, potassium salts such as potassium nitrate, potassium bicarbonate, potassium chloride, potassium citrate, and potassium oxalate; capsaicin; eugenol; strontium salts; zinc salts; chloride salts and combinations thereof. Such agents may be added in effective amounts, e.g., from about 0.5 weight % to about 20 weight % by total weight of the composition, depending on the agent chosen. The compositions defined herein may also be used to treat hypersensitivity by blocking dentin tubules when applied to a tooth surface.

The compositions provided herein may optionally include tartar control agents such as pyrophosphate salts including dialkali or tetraalkali metal pyrophosphate salts such as Na₄P₂O₇, K₄P₂O₇, Na₂K₂P₂O₇, Na₂H₂P₂O₇ and K₂H₂P₂O₇, sodium tripolyphosphate, long chain polyphosphates such as sodium hexametaphosphate and cyclic phosphates such as sodium trimetaphosphate.

The compositions provided herein may further comprise a binder. Any conventional binder may be utilized. Suitable binding agents include marine colloids; carboxyvinyl polymers; carrageenans; starches; cellulosic polymers such as hydroxyethylcellulose. carboxymethylcellulose (carmellose), hydroxypropyl methyl cellulose, and salts thereof (*e*.*g*., carmellose sodium); natural gums such as karaya, xanthan, gum arabic and tragacanth; chitosan; colloidal magnesium aluminum silicate; and colloidal silica. Preferably, a binder is present in the composition in an amount from 0.1 weight % to 5 weight % by total weight of the composition.

Thickening agents which may be incorporated into the compositions defined herein include natural and synthetic gums and colloids. Suitable thickening agents include naturally occurring polymers such as carrageenan, xanthan gum, polyglycols of varying molecular weights sold under the tradename Polyox, and polyvinylpyrrolidone. Compatible inorganic thickening agents include amorphous silica compounds and colloidal silica compounds available under the trade designation Cab-o-sil manufactured by Cabot Corporation. Other inorganic thickening agents include natural and synthetic clays such as hectorite clays, lithium magnesium silicate (laponite) and magnesium aluminum silicate (Veegum).

The compositions defined herein may optionally comprise one or more adhesion agents. The adhesion agent may by a polymeric adherent material. The polymeric adherent material may be any agent that attaches to the surface of a mammalian tooth and/or to a heterogeneous biofilm which also may be present on a tooth's surface. Attachment may occur by any means, such as ionic interaction, van der Waals forces, hydrophobic-hydrophilic interactions, etc. The adherent material may be, for example, any homopolymers or copolymers (hereinafter referred to collectively as a "polymers") that adhere to the surface of a tooth. Such polymers may include cellulose polymers, for example one or more hydroxyalkyl cellulose polymers, such as hydroxypropylmethyl cellulose (HPMC), hydroxyethylpropyl cellulose (HEPC), hydroxybutylmethyl cellulose (HBMC), and carboxymethyl cellulose (CMC). Preferably, the polymeric adherent material comprises at least one cellulose material, for example sodium carboxymethyl cellulose.

The polymeric adherent material may alternatively or additionally include poly (ethylene oxide) polymers (such as POLYOX from Dow Chemical), linear PVP and cross-linked PVP, PEG/PPG copolymers (such as BASF Pluracare L1220), ethylene oxide (EO) - propylene oxide (PO) block copolymers (such as polymers sold under the trade mark Pluronic available from BASF Corporation), ester gum, shellac, pressure sensitive silicone adhesives (such as BioPSA from Dow-Corning), methacrylates, or mixtures thereof. In one embodiment, a copolymer comprises (PVM/MA). Optionally, the copolymer may be selected from the group consisting of: poly (methylvinylether/maleic anhydride), or poly (methylvinylether/maleic acid), or poly (methylvinylether/maleic acid) half esters, or poly (methylvinylether/maleic acid) mixed salts.

Polymers of any molecular weight may be used, including, for example molecular weights of 50,000 to 500,000 Da, 500,000 to 2,500,000 Da or 2,500,000 to 10,000,000 Da (calculated by either number average or weight average).

The oral care compositions defined herein also may include a foam modulator. Foam modulators typically increase the amount of foam produced, for example, when the oral cavity is brushed using the composition in accordance with the methods defined herein. Illustrative examples of foam modulators that increase the amount of foam include, but are not limited to polyoxyethylene and certain polymers including alginate polymers.

The foaming agent is preferably in the oral care composition in an amount from 0.05 to 0.5 weight %, or from 0.1 to about 0.2 weight % by total weight of the composition.

Polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of from 200,000 to 7,000,000 Da, and preferably from 600,000 to 2,000,000 Da, and more preferably from 800,000 to 1,000,000 Da. Polyox® is the trade name for the high molecular weight polyoxyethylene produced by Union Carbide.

Preferably, the compositions provided herein further comprise at least one pH modifying agent. Such agents include acidifying agents to lower pH, basifying agents to raise pH, and buffering agents to control pH within a desired range. The pH modifying agent preferably comprises a basifying agent and/or a buffering agent. For example, one or more compounds selected from acidifying, basifying and buffering agents can be included to provide a pH of 2 to 10, or in various illustrative embodiments, a pH of 2 to 8, 3 to 9, 4 to 8, 5 to 7, 6 to 10, or 7 to 9. Any orally acceptable pH modifying agent can be used including, without limitation, carboxylic, phosphoric and sulfonic acids, acid salts (e.g., monosodium citrate, disodium citrate, monosodium malate); alkali metal hydroxides such as sodium hydroxide; carbonates such as sodium carbonate, bicarbonates, and sesquicarbonates; borates; silicates; phosphates (e.g., monosodium phosphate, trisodium phosphate, pyrophosphate salts), imidazole and the like. One or more pH modifying agents are preferably present in a total amount effective to maintain the composition in an orally acceptable pH range.

Mouth-feel agents that may be incorporated into the compositions used in the methods defined herein include materials which impart a desirable texture or other feeling during use of the composition. Such agents include bicarbonate salts, which may impart a "clean feel" to teeth and gums due to effervescence and release of carbon dioxide. Any orally acceptable bicarbonate can be used, including, without limitation, alkali metal bicarbonates such as sodium and potassium bicarbonates, ammonium bicarbonate, and mixtures thereof. One or more bicarbonate salts are optionally present in a total amount of from 0.1 weight % to 20 weight %, for example from 1% to 15 weight %, by total weight of the composition.

The compositions provided herein may optionally comprise a sweetener. Sweeteners which may be used in the compositions of the present invention include artificial sweeteners such as saccharin, acesulfam, neotam, cyclamate or sucralose; natural high-intensity sweeteners such as thaumatin, stevioside or glycyrrhizin; or sugar alcohols such as sorbitol, xylitol, maltitol or mannitol. These may be present in an amount of up to 0.5 weight %, optionally from 0.005 weight % to 0.1 weight %, based on the total weight of the composition.

The compositions provided herein may optionally comprise a flavorant. Flavorants that may be used in the compositions of the present invention include essential oils as well as various flavoring aldehydes, esters, alcohols, and similar materials. Examples of the essential oils include oils of spearmint, peppermint, aniseed, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint and spearmint. The flavourant may be incorporated in the composition in an amount of from 0.1 weight % to 5 weight %, or from 0.5 weight % to 1.5 weight %, by total weight of the composition.

The compositions provided herein may comprise at least one colorant. Colorants herein include pigments, dyes and agents imparting a particular luster or reflectivity such as pearling agents. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride and the like. One or more colorants are optionally present in a total amount of from 0.001 weight % to about 20 weight %, for example, from 0.01 weight % to 10 weight %, or from 0.1 weight % to 5 weight %, by total weight of the composition.

Preservatives, such as chlorhexidine, triclosan, quaternary ammonium compounds (such as benzalkonium chloride) or parabens (such as methyl or propyl paraben) may be incorporated in the compositions used in the methods of the present invention. The amount of preservative is typically up to 0.5 weight %, optionally from 0.05 to 0.1 weight %, by total weight of the composition.

Preferably, the compositions defined herein comprise a fluoride ion source. Fluoride ion sources include, but are not limited to: stannous fluoride, sodium fluoride, potassium fluoride, potassium monofluorophosphate, sodium monofluorophosphate, ammonium monofluorophosphate, sodium fluorosilicate, ammonium fluorosilicate, amine fluoride such as olaflur (N'-octadecyltrimethylendiamine-Nionic,N,N'-tris(2-ethanol)-dihydrofluoride), ammonium fluoride, and combinations thereof. Optionally, the fluoride ion source includes stannous fluoride, sodium fluoride, amine fluorides, sodium monofluorophosphate, as well as mixtures thereof. Preferably, the oral care composition of the invention may also contain a source of fluoride ions or fluorine-providing ingredient in amounts sufficient to supply about 50 to about 5000 ppm fluoride ion, e.g., from about 100 to about 1000, from about 200 to about 500, or about 250 ppm fluoride ion. Fluoride ion sources may be added to the compositions used in the invention in an amount of from 0.001 weight % to 10 weight %, e.g., from 0.003 weight % to 5 weight %, or from 0.01 weight % to 1 weight % or to 0.05 weight %. However, it is to be understood that the weights of fluoride salts to provide the appropriate level of fluoride ion will vary based on the weight of the counter ion in the salt, and one of skill in the art may readily determine such amounts. A preferred fluoride salt may be sodium fluoride.

In a preferred embodiment, the composition is a dentifrice composition comprising:
40 weight % to 60 weight % humectant, optionally glycerin;
0.1 weight % to 0.4 weight % cellulose binder, optionally sodium carboxymethyl cellulose;
0.05 weight % to 0.4 weight % sweetener, optionally sodium saccharin;
2 weight % to 4 weight % pH modifier, optionally citric acid monohydrate and trisodium citrate dihydrate;
15 weight % to 25 weight % abrasive silica;
1 weight % to 3 weight % thickener silica;
2 weight % to 4 weight % thickener, optionally Polyvinylpyrrolidone;
5 weight % to 10 weight % water;
0.8 weight % to 1.0 weight % sodium methyl cocoyl taurate;
and optionally, 0.5 weight % to 1 weight % poloxamer.

Abrasive silicas are distinct from thickening silicas. In general, abrasive (cleaning) silicas can be characterized as having oil absorption levels of about 40 to 150 cc/100g and having an Einlelmer abrasion of 3 or greater mg loss/100,000 revolutions. In contrast, thickening silicas have oil absorption levels of greater than 150 cc/100 g and an Einlelmer abrasion of less than 2 mg loss/100,000 revolutions.

The abrasive silica is optionally a precipitated or hydrated silica having a mean particle size of up to about 20 microns, such as Zeodent 103, 105, 113, 114, 115, or 124 (marketed by J.M. Huber Chemicals Division, Havre de Grace, Md. 21078), or Sylodent 783 (marketed by Davison Chemical Division of W.R. Grace & Company). Other possible abrasive silicas include silica gels and precipitated amorphous silica having an oil absorption value of less than 100 cc/100 g silica, optionally 45 cc/100 g to less than about 70 cc/100 g silica. These silicas are colloidal particles having an average particle size ranging from about 3 microns to about 12 microns, and optionally between about 5 to about 10 microns.

Thickening silicas include Zeodent 163 (having an oil absorption levels of 190 cc/100g and an Einlelmer abrasion of less than 2 mg loss/100,000 revolutions), Zeodent 165 (having an oil absorption level of 220 cc/100g and having an Einlelmer abrasion of less than 2 mg loss/100,000 revolutions), Zeodent 167 (oil absorption levels of 235 cc/100g and having an Einlelmer abrasion of less than 2 mg loss/100,000 revolutions) (marketed by J.M. Huber Chemicals Division, Havre de Grace, Md. 21078).

### Methods of Use

The present inventors have found that sodium methylcocoyl taurate surfactant, optionally in combination with poloxamer, does not have any irritating effect on the oral mucosa. Therefore, sodium methylcocoyl taurate surfactant, optionally in combination with poloxamer, may advantageously be incorporated into oral care compositions comprising an antioxidant, without counteracting the antioxidant benefit of the compositions.

Accordingly, in one arrangement, provided herein is an oral care composition comprising an antioxidant and a surfactant system, wherein the surfactant system comprises sodium methyl cocoyl taurate in an amount of from 0.7 weight % to 1.2 weight % by total weight of the composition and optionally, a poloxamer in an amount of from 0.4 to 1.2 weight % by total weight of the composition, and wherein the composition is for use in preventing periodontal disease.

The composition may be as defined herein. In a preferred embodiment, the use comprises preventing gingivitis and/or gum receding.

In a further arrangement, provided herein is a use of a surfactant system in an oral care composition comprising an antioxidant, for preserving an antioxidant effect of the composition, wherein the surfactant system comprises sodium methyl cocoyl taurate and optionally, poloxamer, wherein the oral care composition comprises sodium methyl cocoyl taurate in an amount of from 0.7 weight % to 1.2 weight % by total weight of the composition, and optionally, poloxamer in an amount of from 0.4 to 1.2 weight % by total weight of the composition. The antioxidant effect of the composition is provided by the antioxidant.

The composition may be as defined herein. In a preferred embodiment, antioxidant effect of the composition comprises an oral mucosal cell protection effect.

Further provided is a method of preserving an antioxidant effect of an oral care composition comprising an antioxidant, comprising incorporating into the composition a surfactant system comprising sodium methyl cocoyl taurate and optionally, poloxamer, wherein the oral care composition comprises sodium methyl cocoyl taurate in an amount of from 0.7 weight % to 1.2 weight % by total weight of the composition, and optionally, poloxamer in an amount of from 0.4 to 1.2 weight % by total weight of the composition. The antioxidant effect is preserved to a greater extent than that would be observed when using an alternative surfactant such as an alkyl sulfate surfactant.

The composition may be as defined herein. In a preferred embodiment, antioxidant effect of the composition comprises an oral mucosal cell protection effect.

The following Examples illustrate methods of the invention and their uses. The Examples are illustrative and do not limit the scope of the invention.

### EXAMPLES

### Example 1 -Effect of surfactants on oral mucosal cells (1)

Aqueous solutions of different concentrations of sodium lauryl ether sulfate (SLS) and sodium methyl cocoyl taurate (SMCT) were prepared. The solutions were used to challenge oral mucosal cells. Treated cells were stained with Janus Green B and morphological changes were observed using an optical microscope to determine whether or not the cells had become irritated or remained intact. Cells were identified as normal if the cells were in tact, the cell wall was smooth, the color of the cell core was clear/dark, and if organelles could be identified. Cells were identified as irritated if the cells could not be clearly defined with a blurring of the cell wall, if there was crimping of the cell, if the color of the cell core and plasma is lighter than observed for an intact cell, and if organelles could not be identified. The results are illustrated in Table 1.

**Table 1 - Cell irritating effects of aqueous surfactant solutions**

| Surfactant | % active weight surfactant | Results |
|---|---|---|
| SLS | 0.48 | No cell irritation |
| SLS | 1.40 | Cells irritated |
| SLS | 1.71 | Cells irritated |
| SLS | 2.20 | Cells irritated |
| SMCT | 1.40 | No cell irritation |
| SMCT | 2.20 | No cell irritation |

As seen in Table 1, SMCT does not have a cell irritating effect as compared to SLS at comparable concentrations. Whilst 0.48% SLS does not have any cell irritating effects, such a low concentration of surfactant does not provide sufficient foam.

### Example 2 - Irritating effect of surfactants on oral mucosal cells (2)

The method of Example 1 was repeated using SLS/SMCT in toothpaste formulations as illustrated in Table 2.

**Table 2 - Cell irritating effects of SLS and SMCT in toothpaste formulations**

| Ingredients | A | B |
|---|---|---|
| Glycerin | 57.0000 | 57.0000 |
| Sodium CMC - Type 12 | 0.3000 | 0.3000 |
| Sodium Saccharin | 0.2000 | 0.2000 |
| Sodium Fluoride | 0.2200 | 0.2200 |
| Citric Acid Monohydrate | 0.6000 | 0.6000 |
| Trisodium Citrate Dihydrate | 3.0000 | 3.0000 |
| Abrasive Silica | 20.0000 | 20.0000 |
| Thickener Silica | 1.5000 | 1.5000 |
| Flavor | 1.0000 | 1.0000 |
| Sodium Lauryl Sulfate* | 0.5000 | 0.5000 |
| Sodium Methyl Cocoyl Taurate * | 4.1000 | 5.7000 |
| Polyvinylpyrrolidon | 2.0000 | 2.0000 |
| PEG40 Hydrogenated Castor Oil | 2.0000 | 2.0000 |
| Demineralized water | 7.5800 | 5.9800 |
| **Total** | 100.0000 | 100.0000 |

| | | |
|---|---|---|
| * The active level of SLS is 95%. 0.5% is equal to 0.48% active level. The active level of Sodium Methyl Cocoyl Taurate is 30%. 4.1%, 5.7% usage level is equal to 1.23% and 1.71% active level, respectively. | | |

Formulation A comprising 0.48% SLS and 1.23% SMCT showed cell irritating effects according to the criteria described in Example 1. Formulation B comprising 0.48% SLS and 1.71% SMCT caused severe cell irritation.

It could be concluded that the combination of SLS and SMCT was not suitable in oral care formulations, despite good foaming.

### Example 3 - Irritating effect of SMCT on oral mucosal cells

The method of Example 1 was repeated using varying levels of SMCT in toothpaste formulations as illustrated in Table 3.

**Table 3 - Cell irritating effects of SMCT in toothpaste formulations**

| Ingredients | Formula C | Formula D | Formula E |
|---|---|---|---|
| Glycerin | 57.0000 | 57.0000 | 57.0000 |
| Sodium CMC - Type 12 | 0.3000 | 0.3000 | 0.3000 |
| Sodium Saccharin | 0.2000 | 0.2000 | 0.2000 |
| Sodium Fluoride | 0.2200 | 0.2200 | 0.2200 |
| Citric Acid Monohydrate | 0.6000 | 0.6000 | 0.6000 |
| Trisodium Citrate Dihydrate | 3.0000 | 3.0000 | 3.0000 |
| Abrasive Silica | 20.0000 | 20.0000 | 20.0000 |
| Thickener Silica | 1.5000 | 1.5000 | 1.5000 |
| Flavor | 1.0000 | 1.0000 | 1.0000 |
| Sodium Methyl Cocoyl Taurate * | 2.9000 | 5.0000 | 5.7000 |
| Polyvinylpyrrolidon | 2.0000 | 2.0000 | 2.0000 |
| PEG40 Hydrogenated Castor Oil | 2.0000 | 2.0000 | 2.0000 |
| Demineralized water | 9.2800 | 7.1800 | 6.4800 |
| **Total** | 100.0000 | 100.0000 | 100.0000 |
| | | | |
| **Effect on cells** | **No irritation** | **Cells irritated** | **Cells irritated** |

| | | | |
|---|---|---|---|
| *The active level of Sodium Methyl Cocoyl Taurate is 30%. 2.9%, 5.0%, and 5.7% usage level is equal to 0.87%, 1.5%, and 1.71% active level, respectively. | | | |

It can be seen from Table 3 that toothpaste formulations comprising 0.87% SMCT did not have any cell irritating effects. However, when the concentration of SMCT was increased to 1.5% and 1.71%, irritating effects on cells were observed.

The foaming performance of Formulation C comprising 0.87% SMCT was compared with that of a comparable formulation comprising 1.71% SLS using a standard foaming test (Ross Mile Method). As seen in Table 4, the foam volume produced by Formulation C was slightly lower than that produced by the current SLS formulation. However, foaming levels were still acceptable.

**Table 4 - results of foam test**

| Surfactant | Foam production (mm) |
|---|---|
| 1.71% SLS | 130 |
| 0.87% SMCT | 115 |

### Example 4 - Effect of SMCT/Poloxamer 407 on oral mucosal cells

Poloxamer 407 is a nonionic surfactant, with very low cell irritation effect. Since Poloxamer 407 had only a very low cell irritation effect and it was intended to be used in combination with other anionic surfactants, the cell irritating effects of pure Poloxamer 407 solution were not tested. A surfactant system comprising the specific combination of SMCT and Poloxamer 407 was studied, and the cell irritating effect of toothpaste formulations comprising different levels of combined SMCT/Poloxamer 407 were tested by the method described for Example 1. The formulations tested and the results of the tests are illustrated in Table 5 below.

**Table 5 - Cell irritating effects of SMCT/poloxamer in toothpaste formulations**

| Ingredient | Formulation F | Formulation G |
|---|---|---|
| Glycerin | 48.7800 | 48.7800 |
| Sodium CMC - Type 12 | 0.3000 | 0.3000 |
| Sodium Saccharin | 0.2000 | 0.2000 |
| Sodium Fluoride | 0.2200 | 0.2200 |
| Citric Acid Monohydrate | 0.6000 | 0.6000 |
| Trisodium Citrate Dihydrate | 3.0000 | 3.0000 |
| Abrasive Silica | 20.0000 | 20.0000 |
| Thickener Silica | 1.5000 | 1.5000 |
| Flavor | 1.0000 | 1.0000 |
| Sodium Methyl Cocoyl Taurate * | 2.9000 | 2.9000 |
| Poloxamer | 0.5000 | 1.0000 |
| Polyvinylpyrrolidon | 2.0000 | 2.0000 |
| PEG40 Hydrogenated Castor Oil | 2.0000 | 2.0000 |
| Demineralized water | 17.0000 | 16.5000 |
| **Total** | 100.0000 | 100.0000 |
| | | |
| **Effect on cell** | **No irritation** | **No irritation** |

| | | |
|---|---|---|
| *The active level of Sodium Methyl Cocoyl Taurate is 30%. 2.9% usage level is equal to 0.87% active level. | | |

It can be seen from Table 5 that both formulations comprising 0.87% SMCT and poloxamer 407 in an amount of 0.5% or 1%, did not show any cell irritating effects. The foam production of these formulations was also tested and the results are indicated in Table 6 below.

**Table 6 - Results of foam test**

| Surfactant | Foam production |
|---|---|
| 1.71% SLS | 130 |
| 0.87% SMCT and 0.5% poloxamer | 100 |
| 0.87% SMCT and 1% poloxamer | 110 |

As can be seen from Table 6, the foam production of the compositions of the present invention was comparable to that of the corresponding formulation comprising SLS.

### Example 5 - Antioxidant activity of rutin (1)

The antioxidant activity of rutin was tested using an LPO-CC kit (Kamiya Biomedical Company), and compared to that of vitamin E. (The agents were tested in an amount of 1% in ethanol). The results are illustrated in Tables 7a and 7b below. The lower the optical density, the better the antioxidant capacity. The higher the percentage cumeme hydroperoxide reduction, the better the antioxidant capacity.

**Table 7a - antioxidant activity of rutin and vitamin E**

| Agent | Optical Density at 675nm |
|---|---|
| EtOH | 0.4826 |
| 1% rutin | 0.2864 |
| 1% Vitamin E | 0.2204 |

**Table 7b - antioxidant activity of rutin and vitamin E**

| Agent | % reduction of cumeme hydroperoxide |
|---|---|
| EtOH | -0.84494 |
| 1% rutin | 35.6517 |
| 1% Vitamin E | 50.4719 |

It can be seen from Tables 7a and 7b that rutin has a strong antioxidant activity.

### Example 6 - antioxidant activity of rutin (2)

Dentifrice formulations comprising rutin (0.3%) or vitamin E (0.3%) were prepared. The formulations comprised: 35 weight % to 60 weight % humectant, 0.1 weight % to 0.4 weight % cellulose binder, 0.05 weight % to 0.4 weight % sweetener, 2 weight % to 4 weight % pH modifier, 15 weight % to 25 weight % abrasive silica, 1 weight % to 3 weight % thickener silica, 2 weight % to 4 weight % thickener, 1 weight % to 2 weight % zinc salts, 0.3 weight % rutin or vitamin E, and water (qs).

The oral environment is aqueous. During brushing, rutin is released from the dentifrice compositions into the aqueous environment. In order to determine the bioavailability of rutin, rutin was extracted with water from the formulation and rutin levels were analyzed by high performance liquid chromatography. The formulation was subsequently aged at either room temperature or at 40°C for 13 weeks, and the rutin levels measured again. The results are illustrated in Table 8.

**Table 8 - rutin recovery**

| Sample | Detected level (%) | % recovery |
|---|---|---|
| 0.3% rutin (initial) | 0.14 | 46.67 |
| 0.3% rutin (aged 13 weeks room temp.) | 0.06 | 20.00 |
| 0.3% rutin (aged 13 weeks 40°C) | 0.07 | 23.33 |

It can be seen from Table 8 that rutin is "bioavailable" in an aqueous environment, even after aging.

The antioxidant efficacy of the formulations was also tested using the methods described in Example 5. The results are illustrated in Table 9a and 9b.

**Table 9a - Antioxidant activity of rutin and vitamin E**

| Amount of rutin | Optical Density at 675nm |
|---|---|
| Control (no rutin) | 0.4920 |
| 0.3% rutin | 0.4259 |
| 0.3% Vitamin E | 0.4519 |

**Table 9b - Antioxidant activity of rutin and vitamin E**

| Amount of rutin | % reduction of cumeme hydroperoxide |
|---|---|
| Control (no rutin) | -10.5618 |
| 0.3% rutin | 4.3146 |
| 0.3% Vitamin E | 1.5393 |

It can be seen from Tables 9a and 9b that toothpaste formulations comprising rutin exhibit stronger antioxidant activity than comparable formations comprising a comparable amount of vitamin E.

## Claims

1. An oral care composition comprising an antioxidant, wherein the antioxidant is rutin in an amount of from 0.2 weight % to 0.8 weight % by total weight of the composition, and a surfactant system, wherein the surfactant system consists of sodium methyl cocoyl taurate in an amount of from 0.8 weight % to 1 weight % by total weight of the composition and optionally, a poloxamer in an amount of from 0.4 weight % to 1.2 weight % by total weight of the composition, and the composition does not comprise any surfactants other than those of the surfactant system.

2. The composition of claim 1, wherein the rutin is present in the composition in an amount of 0.3 weight % to 0.5 weight %, by total weight of the composition.

3. The composition of claim 1, wherein the composition comprises poloxamer in an amount of from 0.5 weight % to 0.8 weight % by total weight of the composition.

4. The composition of any preceding claim, wherein the composition comprises poloxamer 407.

5. The composition of any preceding claim, wherein the composition is a dentifrice.

6. The composition of claim 5, wherein the composition is a dentifrice composition further comprising:
40 weight % to 60 weight % humectant, optionally glycerin;
0.1 weight % to 0.4 weight % cellulose binder, optionally sodium carboxymethyl cellulose;
0.05 weight % to 0.4 weight % sweetener, optionally sodium saccharin; 2 weight % to 4 weight % pH modifier, optionally citric acid monohydrate and trisodium citrate dihydrate;
15 weight % to 25 weight % abrasive silica;
1 weight % to 3 weight % thickener silica;
2 weight % to 4 weight % thickener, optionally polyvinylpyrrolidone;
5 weight % to 10 weight % water; and
wherein poloxamer is present in an amount of from 0.5 weight % to 1 weight % by total weight of the composition.

7. An oral care composition comprising an antioxidant, wherein the antioxidant is rutin in an amount of from 0.2 weight % to 0.8 weight % by total weight of the composition, and a surfactant system, wherein the surfactant system consists of sodium methyl cocoyl taurate in an amount of from 0.8 weight % to 1 weight % by total weight of the composition and optionally, a poloxamer in an amount of from 0.4 to 1.2 weight % by total weight of the composition, and the composition does not comprise any surfactants other than those of the surfactant system and wherein the composition is for use in preventing periodontal disease.

## Patentansprüche

1. Mundpflegezusammensetzung, umfassend ein Antioxidans, wobei das Antioxidans Rutin in einer Menge von 0,2 Gewichts-% bis 0,8 Gewichts-%, bezogen auf das Gesamtgewicht der Zusammensetzung, ist, und ein Tensidsystem, wobei das Tensidsystem aus Natrium-Methylcocoyltaurat in einer Menge von 0,8 Gewichts-% bis 1 Gewichts-%, bezogen auf das Gesamtgewicht der Zusammensetzung, und wahlweise einem Poloxamer in einer Menge von 0,4 Gewichts-% bis 1,2 Gewichts-%, bezogen auf das Gesamtgewicht der Zusammensetzung, besteht und die Zusammensetzung keine anderen Tenside als die des Tensidsystems umfasst.

2. Zusammensetzung nach Anspruch 1, wobei das Rutin in der Zusammensetzung in einer Menge von 0,3 Gewichts-% bis 0,5 Gewichts-%, bezogen auf das Gesamtgewicht der Zusammensetzung, vorliegt.

3. Zusammensetzung nach Anspruch 1, wobei die Zusammensetzung Poloxamer in einer Menge von 0,5 Gewichts-% bis 0,8 Gewichts-%, bezogen auf das Gesamtgewicht der Zusammensetzung, umfasst.

4. Zusammensetzung nach einem beliebigen vorhergehenden Anspruch, wobei die Zusammensetzung Poloxamer 407 umfasst.

5. Zusammensetzung nach einem beliebigen vorhergehenden Anspruch, wobei die Zusammensetzung ein Zahnputzmittel ist.

6. Zusammensetzung nach Anspruch 5, wobei die Zusammensetzung eine Zahnputzmittelzusammensetzung ist, weiterhin umfassend:
40 Gewichts-% bis 60 Gewichts-% Feuchthaltemittel, wahlweise Glycerin;
0,1 Gewichts-% bis 0,4 Gewichts-% Cellulosebindemittel, wahlweise NatriumCarboxymethylcellulose;
0,05 Gewichts-% bis 0,4 Gewichts-% Süßungsmittel, wahlweise Natriumsaccharin;
2 Gewichts-% bis 4 Gewichts-% pH-Modifikator, wahlweise Zitronensäure-Monohydrat und Trinatriumcitrat-Dihydrat;
15 Gewichts-% bis 25 Gewichts-% abrasives Silica;
1 Gewichts-% bis 3 Gewichts-% Silica-Verdickungsmittel;
2 Gewichts-% bis 4 Gewichts-% Verdickungsmittel, wahlweise Polyvinylpyrrolidon;
5 Gewichts-% bis 10 Gewichts-% Wasser; und
wobei Poloxamer in einer Menge von 0,5 Gewichts-% bis 1 Gewichts-%, bezogen auf das Gesamtgewicht der Zusammensetzung, vorliegt.

7. Mundpflegezusammensetzung, umfassend ein Antioxidans, wobei das Antioxidans Rutin in einer Menge von 0,2 Gewichts-% bis 0,8 Gewichts-%, bezogen auf das Gesamtgewicht der Zusammensetzung, ist, und ein Tensidsystem, wobei das Tensidsystem aus Natrium-Methylcocoyltaurat in einer Menge von 0,8 Gewichts-% bis 1 Gewichts-%, bezogen auf das Gesamtgewicht der Zusammensetzung, und wahlweise einem Poloxamer in einer Menge von 0,4 bis 1,2 Gewichts-%, bezogen auf das Gesamtgewicht der Zusammensetzung, besteht und die Zusammensetzung keine anderen Tenside als die des Tensidsystems umfasst und wobei die Zusammensetzung zur Verwendung in der Prävention von Parodontitis ist.

## Revendications

1. Composition de soins bucco-dentaires comprenant un antioxydant, dans laquelle l'antioxydant est la rutine en une quantité de 0,2 % en poids à 0,8 % en poids par rapport au poids total de la composition, et un système tensioactif, dans laquelle le système tensioactif est constitué de méthyl cocoyl taurate de sodium en une quantité de 0,8 % en poids à 1 % en poids par rapport au poids total de la composition et éventuellement, un poloxamère en une quantité de 0,4 % en poids à 1,2 % en poids par rapport au poids total de la composition, et la composition ne comprend pas de tensioactifs autres que ceux du système tensioactif.

2. Composition selon la revendication 1, dans laquelle la rutine est présente dans la composition en une quantité de 0,3 % en poids à 0,5 % en poids, par rapport au poids total de la composition.

3. Composition selon la revendication 1, dans laquelle la composition comprend du poloxamère en une quantité de 0,5 % en poids à 0,8 % en poids par rapport au poids total de la composition.

4. Composition selon l'une quelconque des revendications précédentes, dans laquelle la composition comprend du poloxamère 407.

5. Composition selon l'une quelconque des revendications précédentes, dans laquelle la composition est un dentifrice.

6. Composition selon la revendication 5, dans laquelle la composition est une composition de dentifrice comprenant en outre :
40 % en poids à 60 % en poids d'humectant, éventuellement de la glycérine ;
0,1 % en poids à 0,4 % en poids de liant cellulosique, éventuellement de la carboxyméthylcellulose sodique ;
0,05 % en poids à 0,4 % en poids d'édulcorant, éventuellement de la saccharine sodique ;
2 % en poids à 4 % en poids de modificateur de pH, éventuellement de l'acide citrique monohydraté et du citrate trisodique dihydraté ;
15 % en poids à 25 % en poids de silice abrasive ;
1 % en poids à 3 % en poids de silice épaississante ;
2 % en poids à 4 % en poids d'épaississant, éventuellement de la polyvinylpyrrolidone ;
5 % en poids à 10 % en poids d'eau ; et
dans laquelle le poloxamère est présent en une quantité de 0,5 % en poids à 1 % en poids par rapport au poids total de la composition.

7. Composition de soins bucco-dentaires comprenant un antioxydant, dans laquelle l'antioxydant est la rutine en une quantité de 0,2 % en poids à 0,8 % en poids par rapport au poids total de la composition, et un système tensioactif, dans laquelle le système tensioactif est constitué de méthyl cocoyl taurate de sodium en une quantité de 0,8 % en poids à 1 % en poids par rapport au poids total de la composition et éventuellement, un poloxamère en une quantité de 0,4 à 1,2 % en poids par rapport au poids total de la composition, et la composition ne comprend aucun tensioactifs autres que ceux du système tensioactif et dans laquelle la composition est pour une utilisation dans la prévention d'une maladie parodontale.
